# EUROPÄISCHE PATENTSCHRIFT

(11) **EP 2 299 880 B1**
(45) Veröffentlichungstag und Bekanntmachung des Hinweises auf die Patenterteilung: **23.01.2013**
(21) Anmeldenummer: 09761675.9
(22) Anmeldetag: 08.06.2009
(51) Int. Cl.: A47J 31/00, A47J 31/36

(54) **VERFAHREN UND VORRICHTUNG FÜR VOLUMENSTROMABHÄNGIGEN CREMAERZEUGUNG**
METHOD AND DEVICE FOR CREMA PRODUCTION BASED ON VOLUMETRIC FLOW
PROCÉDÉ ET DISPOSITIF POUR LA PRODUCTION DE MOUSSE EN FONCTION DU FLUX VOLUMIQUE

(30) Priorität: 12.06.2008 DE 102008028031
(43) Veröffentlichungstag der Anmeldung: 30.03.2011
(73) Patentinhaber: BSH Bosch und Siemens Hausgeräte GmbH, 81739 München (DE)
(72) Erfinder: DABURGER, Josef, 83313 Siegsdorf / Hammer (DE); GERL, Ulrike, 83278 Traunstein (DE); MITROVIC, Marijana Jerance, OX2 0EX Oxford, Oxfordshire (GB)
(86) Internationale Anmeldenummer: PCT/EP2009/056997
(87) Internationale Veröffentlichungsnummer: WO 2009/150112

(56) Entgegenhaltungen:
- EP-A- 0 486 434
- EP-A1- 1 440 644
- EP-A1- 1 462 042
- WO-A-2007/076567
- WO-A-2007/110768
- WO-A1-2008/078989
- US-A1- 2007 261 564

## Beschreibung

Die Erfindung betrifft ein Verfahren zur Erzeugung von Crema in einem Kaffeegetränkeautomaten, der einen Durchlauferhitzer zur Erhitzung von Wasser umfasst und der mit Substratkapseln bestückbar ist, die eine Venturi-Düse für die Cremaerzeugung umfassen, mit den folgenden, kontinuierlich ablaufenden Schritten:
a) Erhitzen des Wassers in dem Durchlauferhitzer
b) Aufbrühen von Kaffeemehl mit dem erhitzten Wasser in einer Brühkammer zur Erzeugung eines Kaffeegetränks und
c) Erzeugung von Crema durch Vermischen des Kaffeegetränks mit einem Gas in der Venturi-Düse.

Die Erfindung bezieht sich darüber hinaus auf eine entsprechende Vorrichtung zur Durchführung dieses Verfahrens.

Kaffeegetränke, insbesondere Espressi, erhalten durch den hohen Druck von zwischen 2 und 10 bar, unter den sie hergestellt werden, auf ihrer Oberfläche einen leichten feinen Schaum, die sogenannte Crema. Werden Kaffeegetränke jedoch unter geringerem Druck, typischerweise mit etwa 1 bar hergestellt, treten die druckbasierten physikalischchemischen Prozesse der Espressoherstellung nicht ein. Es kann sich zwar ein Schaum bilden, der jedoch in der Regel weder feinblasig noch dauerhaft ist. Faktoren, die die Cremabildung beeinflussen sind beispielsweise aus der US 2007/0261564 A1 und der WO 2007/110768 A2 bekannt.

Um dennoch eine feinblasige Crema zu erreichen, kann das Kaffeegetränk u. a. durch eine Venturi-Düse geleitet werden. Darin kann die Blasengröße des Schaums relativ exakt eingestellt und eine möglichst homogene Größenverteilung der Schaumblasen erreicht werden. Die so produzierte Crema ist darüber hinaus auch sehr stabil bzw. standfest. Die relevanten Parameter für die Cremaerzeugung in einer Venturi-Düse sind zum einen die Strömungsgeschwindigkeit des Kaffeegetränks durch die Düse und zum anderen der Lochquerschnitt, durch den zur Schaumbildung ein Gas, in der Regel Umgebungsluft, eingesaugt wird. Sowohl die Strömungsgeschwindigkeit als auch der Lochdurchmesser bestimmen die Menge des eingeblasenen Gases.

Es ist ein Mehrgetränkegerät auf dem Markt, das mit Getränkesubstratkapseln bestückbar ist und mit dem neben Tee, Kakao und sogar Suppenbrühen auch Kaffee hergestellt werden kann. Da für die anderen Getränke in der Regel keine Schaumerzeugung gewünscht ist, ist die für die Cremaerzeugung erforderliche Venturi-Düse nicht geräteseitig, sondern in der Kapsel angeordnet. Für eine geräteseitige Verbesserung der Cremabildung kann daher auf die Dimensionierung der Venturi-Düse nicht zurückgegriffen werden. Die EP 1 440 644 A1 offenbart eine derartiges Mehrgetränkegerät wie auch die zugehörigen Getränkesubstratkapseln.

Aufgabe der Erfindung ist daher, ein Verfahren und eine Vorrichtung der eingangs genannten Art anzugeben, bei denen eine bessere Cremabildung erreicht werden kann.

Diese Aufgabe wird erfindungsgemäß dadurch gelöst, dass wenigstens eine der beiden Volumenströme, also der des Kaffeegetränks oder des Gases, in der Venturi-Düse für die Dauer eines bestimmten Zeitintervalls erhöht wird. Die Erfindung verlässt also den naheliegenden Lösungsansatz, zur Verbesserung der Cremabildung vorrichtungsseitig die Dimensionierung der Venturi-Düse zu verändern. Sie verfolgt vielmehr das Prinzip, für eine verbesserte Cremabildung bei unveränderter Venturi-Düse die Volumenströme zu beeinflussen, nämlich zu erhöhen. Es kann entweder die Gas- bzw. Luftzufuhr in dem vorhandenen Strom des Kaffeegetränks erhöht werden, wodurch sich der Anteil an gebildeter Crema erhöht, oder es kann der Volumenstrom des Kaffeegetränks erhöht werden. Die Erfindung nutzt dann die an sich bekannten Prinzipien der Venturi-Düse geschickt für sich, wonach eine erhöhte Strömungsgeschwindigkeit bzw. ein erhöhter Volumenstrom des Fluids durch die Venturi-Düse ein verstärktes Ansaugen von Gas zu Folge hat. Mit der Vergrößerung des Volumenstroms des Kaffeegetränks erhöht sich also zugleich auch der des Gasstroms in der Venturi-Düse.

Eine dauerhafte Erhöhung des bzw. der Volumenströme brächte unterschiedliche Nachteile mit sich. Eine Erhöhung des Kaffeestroms bedeutet zugleich auch einen schnelleren Durchsatz durch den Durchlauferhitzer. Dabei besteht die Gefahr, dass das Zubereitungswasser bei einer im Übrigen unveränderten Auslegung des Getränkegeräts wegen der zu geringen Aufenthaltsdauer im Durchlauferhitzer nicht ausreichend erhitzt wird und das bezogene Kaffeegetränk daher eine geringere Temperatur erhält. Die Erhöhung des Volumenstroms durch die Venturi-Düse wird daher zeitlich beschränkt, so dass eine verminderte Wärmeaufnahme des Wassers sich nicht geschmacklich auswirkt, aber eine bessere oder mehr Crema produziert wird. Erfindungsgemäß ist die Dauer des Zeitintervalls volumenstromabhängig, wird also nach der geförderten Wassermenge bestimmt. Da in dem Zubereitungsgerät ohnehin ein Durchflussmesser zur Abmessung einer Tassenportion vorhanden ist, ist dafür kein nennenswerter zusätzlicher technischer Aufwand erforderlich.

Die Dauer des Zeitintervalls kann nach einer weiteren vorteilhaften Ausgestaltung der Erfindung maschinenseitig fest vorgegeben sein. Dies vereinfacht die Bedienung des Zubereitungsgerätes, weil ein Benutzer des Gerätes keine Aufmerksamkeit auf eine zusätzliche Einstellung, die ihm vielleicht nicht vollständig nachvollziehbar erscheint, richten muss.

Nach einer dazu alternativen Ausgestaltungsform der Erfindung kann das Zeitintervall von einem Benutzer manuell eingestellt werden. Der Benutzer erhält dadurch eine größere Wahlfreiheit und kann das Kaffeegetränk individuell seinem Geschmack und seinen Wünschen nach mehr oder weniger Crema anpassen.

Sowohl für das Ergebnis der Cremaerzeugung als auch für den Geschmack des zubereiteten Kaffeegetränks kann der Zeitpunkt der Volumenstromerhöhung von Bedeutung sein. Erfindungsgemäß erfolgt daher die Erhöhung des wenigstens einen Volumenstroms am Ende eines Tassenzubereitungsvorgangs. Dies hat den Vorteil, dass die Crema gegen Ende des Zubereitungsvorgangs auf das im Übrigen fertig zubereitete Getränk aufgebracht wird, so dass die Crema nicht durch das nachfolgende Getränk wieder zerstört wird. Geschmackliche Auswirkungen kann diese Ausführungsform dadurch zeitigen, dass die Zubereitung des Kaffeegetränks hinsichtlich der Strömungsgeschwindigkeit des Wassers jedenfalls durch den Durchlauferhitzer optimal für die Brühung des Getränks eingestellt werden kann. Denn die ungestörte Idealbrühung in einer ersten Zubereitungsphase einer Tassenportion bewirkt den geschmacklichen Charakter des Getränks, wie er in Mitteleuropa im Allgemeinen bevorzugt wird. Eine Beschleunigung des Volumenstroms zugunsten der Cremabildung erst am Ende des Tassenzubereitungsvorgangs hat daher geringe geschmackliche Auswirkungen gegenüber der derzeitigen Zubereitung.

Nicht zur Erfindung zählt eine Alternative, nämlich die Erhöhung des wenigstens einen Volumenstroms zu Beginn eines Tassenzubereitungsvorgangs. Diese Alternative hat den Vorteil einer besonders stabilen Crema, weil zu Beginn des Eluationsprozesses besonders viele Schaumbildner aus dem Kaffeeextrakt gelöst werden. Dadurch lässt sich ein stabilerer Schaum generieren, der auch durch nachfolgendes Kaffeegetränk im kaum nennenswerten Umfang mehr zerstört wird.

Grundsätzlich kann entweder der Gasstrom oder der Kaffeestrom in der Venturi-Düse erhöht werden. Nach einer weiteren vorteilhaften Ausgestaltung der Erfindung kann die Förderleistung des Wassers über eine Optimalleistung hinaus, bei der das Wasser im Durchlauferhitzer auf die erforderliche Brühtemperatur erhitzt würde, erhöht werden. Mit der Erhöhung der Förderleistung des Wassers, mit der zugleich mehr Kaffeegetränk durch die Venturi-Düse transportiert wird, erhöht sich auch der Gasstrom des angesaugten Gases, in der Regel Umgebungsluft. Mit diesem Verfahren kann das Zubereitungsgerät technisch im Wesentlichen unverändert bleiben. Lediglich die Förderleistung der Pumpe muss für ein bestimmtes Zeitintervall heraufgesetzt werden, zum Beispiel durch Erhöhung der Taktung einer Schwingkolbenpumpe. Insbesondere die Heizleistung des Durchlauferhitzers muss nicht erhöht werden.

Nach einer weiteren vorteilhaften Ausgestaltung der Erfindung kann der Durchlauferhitzer vor jeder Zubereitung einer Tassenportion vorgeheizt werden, ohne dass bereits Wasser gefördert würde. Damit lässt sich der unterbliebene Wärmeeintrag in das Wasser kompensieren, der bei einer beschleunigten Förderung des Wassers zum Zwecke der Creamaerzeugung durch die verkürzte Aufenthaltsdauer des Wassers im Durchlauferhitzer entstehen kann. Somit kann eine negative Auswirkung der Cremaerzeugung auf die Temperatur des Kaffeegetränks vermindert werden.

Nach einer weiteren vorteilhaften Ausgestaltung der Erfindung kann beim Durchströmen der Venturi-Düse mit Kaffeegetränk zusätzlich Gas zugeführt werden. Diese Maßnahme kann allein oder zusätzlich zur Erhöhung des Volumenstroms des Kaffeegetränks ergriffen werden. Dadurch kann eine verbesserte Cremabildung erzielt werden, ohne dass der übrige Kaffeezubereitungsvorgang beispielsweise hinsichtlich Strömungsgeschwindigkeit und Temperatur des Kaffeegetränks beeinflusst würde. Die Cremaerzeugung kann also nahezu vollkommen unabhängig von der Zubereitung einer Tassenportion erfolgen.

Die der Erfindung gestellte Aufgabe kann bei dem eingangs erwähnten Kaffeegetränkeautomaten zur Kaffeezubereitung mit Substratkapseln, die in einer Brühkammer einsetzbar sind und eine Venturi-Düse für eine Gaszufuhr zur Cremaerzeugung umfassen, und der einen Durchlauferhitzer zur Erhitzung von Wasser auf Brühtemperatur und eine Pumpe für den Transport des Wassers vom Durchlauferhitzer in die Brühkammer umfasst, dadurch gelöst werden, dass er über eine Vorrichtung zur Erhöhung wenigstens eines der beiden Volumenströme von Kaffeegetränk oder Gas in der Venturi-Düse für die Dauer eines bestimmten volumenstromabhängig bestimmbaren Zeitintervalls am Ende eines Tassenzubereitungsvorgangs umfasst. Die Ereindung verfolgt also das Prinzip, die Cremabildung ohne Änderung der Venturi-Düse zu beeinflussen bzw. zu verbessern. Erfindungsgemäß gelingt dies geräteseitig, so dass keine Änderung an der Kapsel vorgenommen werden muss.

Erfindungsgemäß ist das Zeitintervall volumenstromabhängig bestimmbar. Dafür sind keine zusätzlichen technischen Einrichtungen erforderlich, weil zur Bestimmung der Menge für eine Tassenportion ohnehin ein Durchflussmesser in dem Kaffeegetränkeautomat vorhanden ist. Die Bestimmung der Dauer des Zeitintervalls, für die der Volumenstrom erhöht wird, bedeutet also keinen zusätzlichen technischen Aufwand und damit keine zusätzlichen Kosten.

Die Dauer des Zeitintervalls kann maschinenseitig fest vorgegeben bzw. voreingestellt sein. Nach einer weiteren vorteilhaften Ausgestaltung der Erfindung kann die Dauer des Zeitintervalls manuell bestimmbar sein. Damit kann dem Benutzer des Kaffeegetränkeautomaten eine Wahlmöglichkeit geboten werden, sein Kaffeegetränk individuell seinen Neigungen und seinem Geschmack anzupassen. Der Getränkeautomat erhält dadurch eine attraktive Zusatzfunktion.

Nach einer weiteren vorteilhaften Ausgestaltung der Erfindung kann der Kaffeegetränkeautomat über eine Steuerungseinrichtung verfügen. Mit dieser Vorrichtung kann die Förderleistung der Pumpe für die bestimmte Zeitdauer über eine Optimalleistung hinaus, bei der das Wasser auf die erforderliche Brühtemperatur erhitzt würde, erhöht werden. Die Steuerungseinrichtung schaltet also die Pumpe zu der erforderlichen Zeit und für das erforderliche Zeitintervall auf eine höhere Leistung. Bei einer volumenstromabhängigen Bestimmung des Zeitintervalls empfängt und verarbeitet sie Signale eines Durchflusszählers, der für die Bemessung einer Tassenportion ohnehin erforderlich ist. Bei einer rein zeitgestützten Steuerung kann sie Signale aus einem separaten Zeitglied verarbeiten.

Bei dieser Ausführungsform wird also der Volumenstrom des Kaffeegetränks durch die Venturi-Düse erhöht. Den physikalischen Gesetzen der Venturi-Düse folgend erhöht sich dadurch auch der Gasstrom, der durch den Fluidstrom des Kaffeegetränks angesogen wird. Durch diese Ausführungsform der Erfindung kann also die Cremabildung des Getränkeautomaten verbessert werden, ohne dass große technische Änderungen hinsichtlich der Leistung der einzelnen Komponenten, insbesondere der Heizung oder der Venturi-Düse vorgenommen werden müssten. Lediglich die Pumpe muss für eine temporäre Leistungssteigerung, beispielsweise für eine vorübergehende erhöhte Taktung einer Schwingkolbenpumpe, ausgelegt sein.

Der Volumenstrom des Kaffeegetränks in der Venturi-Düse ist mit dem Volumenstrom des Wassers in der Heizeinrichtung so gut wie identisch, denn beide Volumenströme werden von derselben Pumpe gefördert. Der Wasserstrom ist auf eine optimale Verweildauer des Wassers im Durchlauferhitzer eingestellt, die für seine Erhitzung zur Zubereitung des Kaffeegetränks erforderlich ist. Eine Erhöhung des Volumenstroms in der Venturi-Düse zieht also auch eine Erhöhung des Volumenstroms im Durchlauferhitzer nach sich. Damit verkürzt sich die Verweildauer des Wassers im Durchlauferhitzer, folglich verringert sich seine Energieaufnahme. Das Wasser wird also weniger erhitzt. Daher kann nach einer weiteren vorteilhaften Ausgestaltung der Erfindung die Steuerungseinrichtung dafür ausgelegt sein, dass der Durchlauferhitzer vor der Zubereitung jeder Tassenportion vorgeheizt wird, ohne dass sich bereits Wasser im Durchlauferhitzer befindet. Dadurch kann eine sonst verringerte Temperaturaufnahme infolge der höheren Durchströmgeschwindigkeit für eine verbesserte Cremaerzeugung nahezu kompensiert werden.

Nach einer dazu alternativen Ausgestaltungsform der Erfindung kann der Getränkeautomat über eine Fördereinrichtung für die Gaszufuhr verfügen. Dies stellt zwar einen zusätzlichen technischen Aufwand dar, hat aber den entscheidenden Vorteil, dass die technische Ausstattung des Getränkeautomaten im Übrigen unverändert bleiben kann. Eine Beeinflussung des Brühvorgangs insbesondere mit geschmacklichen Auswirkungen muss daher nicht befürchtet werden. Die Gaszufuhr kann zudem vollkommen unabhängig vom übrigen Getränkezubereitungsvorgang gesteuert werden, womit sie für eine separate manuelle Einstellung prädestiniert ist. Der Benutzer kann damit auf sehr komfortable Weise die Cremaerzeugung seinen Wünschen anpassen, ohne geschmackliche oder temperaturbezogene Nachteile seines Getränks in Kauf nehmen zu müssen. Die Fördereinrichtung für die Gaszufuhr kann zum Beispiel eine zusätzliche Pumpe für Umgebungsluft umfassen oder sich aus Kartuschen mit komprimiertem Gas bzw. Kohlendioxid speisen, die im Handel für andere Anwendungen bereits erhältlich sind.

Das Prinzip der Erfindung wird nachfolgend anhand einer Zeichnung beispielshalber noch näher erläutert. In der Zeichnung zeigen:
- Figur 1:: einen schematischen Aufbau einer Kaffeezubereitungsmaschine,
- Figur 2:: einen Temperatur- und einen Förderverlauf nach einer ersten Steuerungsmöglichkeit, und
- Figur 3:: die Verläufe nach einer zweiten Steuerungsmöglichkeit.

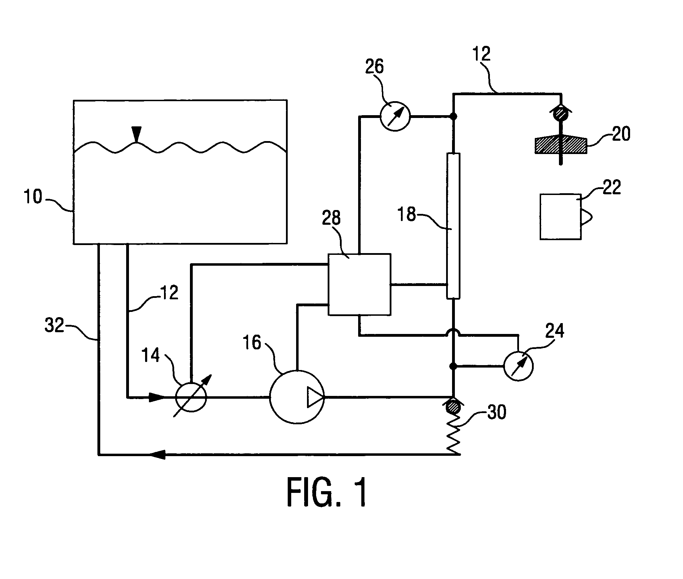

Figur 1 stellt die wesentlichen Bestandteile eine Kaffeezubereitungsmaschine schematisch dar. Von einem Frischwassertank 10 verläuft eine Leitung 12 zu einem Durchflusszähler 14, der über die Leitung 12 im Weiteren mit einer Pumpe 16 und einem Durchlauferhitzer 18 als Heizung verbunden ist. Stromab des Durchlauferhitzers 18 mündet die Leitung 12 in eine Brühkammer 20, in die eine nicht dargestellte Getränkekapsel mit einer Venturi-Düse einsetzbar ist. Das dort zubereitete Getränk gelangt in eine Tasse 22 stromab der Brühkammer 20. Jeweils stromauf und stromab des Durchlauferhitzers 18 ist jeweils ein Temperatursensor 24, 26 angeordnet, der die Wassertemperatur eingangs bzw. ausgangs des Durchlauferhitzers 18 erfasst. Die Temperatursensoren 24, 26 sowie die Pumpe 16 und der Durchflusszähler 14 sind mit einer Steuerungseinrichtung 28 elektrisch gekoppelt. Sie empfängt und verarbeitet Signale des Durchflusszählers 14 und der Temperatursensoren 24, 26 und steuert die Pumpe 16 und den Durchlauferhitzer 18. Zwischen der Pumpe 16 und dem Temperatursensor 24 ist ein Überdruckventil 30 angeordnet, das Überdruck über eine Entlastungsleitung 32 in den Frischwassertank 10 zurückleitet.

Zur Zubereitung eines Kaffeegetränks fördert die Pumpe 16 kontinuierlich Frischwasser aus dem Wassertank 10 über die Leitung 12 in den Durchlauferhitzer 18. Das dort erhitzte Wasser gelangt durch Nachströmen weiteren Frischwassers in die Brühkammer 20. Dort wird es in eine Kapsel mit Getränkekonzentrat oder Kaffeepulver injiziert und zu einem Kaffeegetränk gebrüht. Am Auslauf der Kapsel strömt das Kaffeegetränk durch eine Venturi-Düse, in der es Umgebungsluft ansaugt und mit ihr vermischt wird. Das Kaffeegetränk wird in die Tasse 22 abgegeben und bildet auf seiner Oberfläche eine feine Crema.

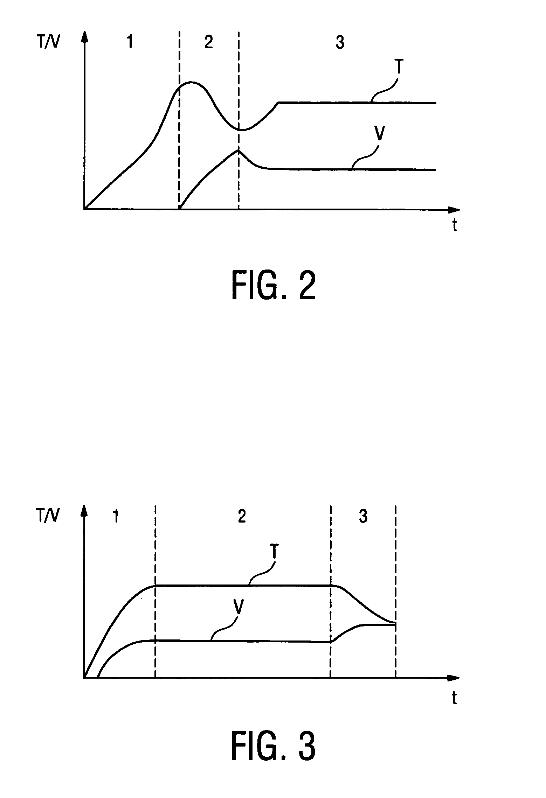

Die vorliegende Kaffeemaschine verfügt mit der Steuereinrichtung 28 über eine Vorrichtung, mit der der Volumenstrom des Kaffeegetränks durch die Venturi-Düse erhöht werden kann, um ohne Veränderungen an der Venturi-Düse selbst bessere bzw. mehr Crema zu erzeugen. Die zeitlich beschränkte Erhöhung des Volumenstroms kann grundsätzlich zu einem beliebigen Zeitpunkt während des gesamten Zubereitungsvorgangs vorgenommen werden. In den Figuren 2 und 3 sind zwei Verläufe der Temperatur T und des Volumenstroms V während der Zeit t qualitativ dargestellt für eine Erhöhung des Volumenstroms V des Wassers zu Beginn des Zubereitungsvorgangs (Figur 2) und an seinem Ende (Figur 3).

Bei einer Erhöhung des Volumenstroms V zu Beginn des Zubereitungsvorgangs nach Figur 2 setzt die Steuerungseinrichtung 28 nach Einschalten des Getränkeautomaten bzw. nach einer Anforderung einer Tassenportion nicht wie bisher mehr oder weniger sofort die Pumpe 16 in Betrieb, sondern heizt zunächst in einer Phase 1 den Durchlauferhitzer 18 vor. Erst in einer anschließenden Phase 2 setzt die Steuerungseinrichtung 28 die Pumpe 16 in Gang. Ihre Förderleistung ist dabei zunächst so bemessen, dass die geförderte Wassermenge für die korrekte Getränkezubereitung in einem nicht vorgeheizten Durchlauferhitzer 18 nicht genug erhitzt würde. Der dadurch an sich zu geringe Wärmeeintrag wird durch die Vorheizung des Durchlauferhitzers 18 in der Phase 1 zumindest teilweise kompensiert.

Aufgrund der hohen Strömungsgeschwindigkeit des Frischwassers durch den Durchlauferhitzer 18 fällt dessen Temperatur während der Phase 2 gegenüber seiner normalen Betriebstemperatur während eines Zubereitungsvorganges ab. In der Phase 2 wird wegen der erhöhten Strömungsgeschwindigkeit des Frischwassers bzw. des Kaffeegetränks durch die Venturi-Düse besonders viel feinblasige Crema erzeugt. Sie ist zudem besonders stabil und haltbar, da zu Beginn der Kaffeeextraktikon besonders konzentriert oberflächenaktive Moleküle aus dem Kaffeepulver der Getränkekapsel extrahiert werden

Nach einem gewissen Fördervolumen, zum Beispiel nach einer Pumpleistung von 50 ml, wird die Fördergeschwindigkeit in einer Phase 3 auf ein Normalmaß verringert, das für eine optimale Getränkezubereitung geeignet ist. Durch den geringeren Durchsatz an Frischwasser durch den Durchlauferhitzer 18 steigt seine Temperatur auf die Zubereitungstemperatur für ein Kaffeegetränk wieder an. In der Phase 3 wird dann unter den nun herrschenden Normalverhältnissen das übrige Kaffeegetränk zubereitet, bis eine Tassenportion erstellt ist.

Eine alternative Steuerungsmöglichkeit zeigt die Figur 3. Demnach wird zunächst in einer Phase 1 wie herkömmlich nach Einschalten des Getränkeautomaten bzw. nach Anforderung einer Tassenportion der Durchlauferhitzer 18 und wenig später auch die Pumpe 16 eingeschaltet, um Brühwasser für die Getränkezubereitung bereitzustellen. In der Phase 2 geben sowohl die Fördergeschwindigkeit als auch die Temperatur des Durchlauferhitzers 18 die optimalen Zubereitungsverhältnisse wieder. In dieser Phase wird daher der Hauptteil der Tassenportion hergestellt.

In einer abschließenden Phase 3 steigert die Steuerungseinrichtung 28 die Leistung der Pumpe 16, so dass durch den jetzt höheren Wasserdurchsatz durch die Heizung 18 deren Temperatur geringfügig abfällt. Die höhere Pumpleistung lässt das fertige Kaffeegetränk mit erhöhter Geschwindigkeit durch die Venturi-Düse strömen und dort vermehrt Umgebungsluft ansaugen. Erst in der Phase 3 wird also verstärkt feinblasige Crema erzeugt, die nach Abschluss des eigentlichen Zubereitungsvorgangs einer Tassenportion auf deren Oberfläche eingeschenkt wird. Da dieser Schritt den Abschluss des Zubereitungsvorgangs darstellt, kann die Crema nicht durch nachfolgendes weiteres Kaffeegetränk wieder zerstört werden. Diese Steuerung stellt darüber hinaus nur eine geringe Abwandlung gegenüber einer herkömmlichen Steuerung des Getränkeautomaten dar. Insbesondere die geschmacksrelevanten Phasen 1 und 2 am Anfang des Zubereitungsvorgangs sind unverändert bzw. ungestört. Das geschmackliche Ergebnis ist durch dieses Zubereitungsverfahren von Crema daher so gut wie nicht beeinflusst.

Das zuletzt dargestellte Zubereitungsverfahren ist also der herkömmlichen, auf den mitteleuropäischen Kaffeegeschmack abgestimmten Zubereitung am ähnlichsten. Aber auch die erste Verfahrensvariante hat ihre Berechtigung, wenn das Interesse eher auf einer verstärkten Cremabildung liegt oder der dabei erzielte herbere Geschmack bevorzugt wird.

### Bezugszeichenliste:

- 10: Frischwassertank
- 12: Flüssigkeitsleitung
- 14: Durchflusszähler
- 16: Pumpe
- 18: Durchlauferhitzer
- 20: Brühkammer mit Kapsel und Venturi-Düse
- 22: Tasse
- 24,26: Temperatursensor
- 28: Steuerungseinrichtung
- 30: Überdruckventil
- 32: Entlastungsleitung

- T: Temperatur
- V: Volumenstrom

## Patentansprüche

1. Verfahren zur Erzeugung von Crema in einem Kaffeegetränkeautomaten, der einen Durchlauferhitzer (18) zur Erhitzung von Wasser umfasst und der mit Substratkapseln bestückbar ist, die eine Venturi-Düse für die Cremaerzeugung umfasst, mit den folgenden, kontinuierlich ablaufenden Schritten:
a) Erhitzen des Wasser in dem Durchlauferhitzer (18),
b) Aufbrühen von Kaffeemehl mit dem erhitzten Wasser in einer Brühkammer (20) zur Erzeugung eines Kaffeegetränks,
c) Erzeugen von Crema durch Vermischen des Kaffeegetränks mit einem Gas in der Venturi-Düse,
**dadurch gekennzeichnet, dass** in Schritt c) wenigstens einer der beiden Volumenströme (V) von Kaffeegetränk oder Gas in der Venturi-Düse für die Dauer eines bestimmten durchflussabhängig gesteuerten Zeitintervalls am Ende eines Tassenzubereitungsvorgangs erhöht wird.

2. Verfahren nach Anspruchs 1, **dadurch gekennzeichnet, dass** die Förderleistung des Wassers über eine Optimalleistung hinaus, bei der das Wasser im Durchlauferhitzer (18) auf die erforderliche Brühtemperatur erhitzt würde, erhöht wird.

3. Verfahren nach obigem Anspruch, **dadurch gekennzeichnet, dass** der Durchlauferhitzer (18) zu Beginn eines Zubereitungsvorgangs einer Tassenportion vor der Förderung von Wasser zum Erhitzen im Schritt a) vorgeheizt wird.

4. Verfahren nach einem der obigen Ansprüche, **dadurch gekennzeichnet, dass** beim Durchströmen der Venturi-Düse in Schritt c) zusätzlich Gas gefördert wird.

5. Kaffeegetränkeautomat zur Kaffeezubereitung aus Substratkapseln, die in eine Brühkammer (20) einsetzbar sind und eine Venturi-Düse mit einer Gaszufuhr zur Cremaerzeugung umfassen, mit einem Durchlauferhitzer (18) zur Erhitzung von Wasser auf Brühtemperatur, mit einer Pumpe (16) für den Transport des Wassers von einem Vorratstank (10) über den Durchlauferhitzer (18) in die Brühkammer (20), **gekennzeichnet durch** eine Vorrichtung (28) zur Erhöhung wenigstens einer der beiden Volumenströme (V) von Kaffeegetränk oder Gas in der Venturi-Düse für die Dauer eines bestimmten volumenstromabhängig bestimmbaren Zeitintervalls am Ende eines Tassenzubereitungsvorgangs.

6. Kaffeegetränkeautomat nach Anspruch 5, **gekennzeichnet durch** eine Steuerungseinrichtung (28) als Vorrichtung, mit der die Förderleistung der Pumpe (16) für die bestimmte Dauer über eine Optimalleistung hinaus, bei der das Wasser auf die erforderliche Brühtemperatur erhitzt wird, erhöht werden kann.

7. Kaffeegetränkeautomat nach einem der Ansprüche 5 bis 6, **gekennzeichnet durch** eine Fördereinrichtung für die Gaszufuhr.

## Claims

1. Method of producing foam in a coffee drinks machine, which comprises a throughflow heater (18) for heating water and which can be furnished with substrate capsules comprising a venturi nozzle for producing foam, with the following continuously sequential steps:
a) heating the water in the throughflow heater (18),
b) brewing coffee grinds with the heated water in a brewing chamber (20) for producing a coffee beverage and
c) producing foam by mixing the coffee beverage with a gas in the venturi nozzle,
**characterised in that** in step c) at least one of the two volume flows (V) of coffee beverage or gas in the venturi nozzle is increased for the duration of a specific time interval, which is controlled in dependence on throughflow, at the end of a cup preparation process.

2. Method according to claim 1, **characterised in that** the conveying output of the water is increased beyond an optimum output at which the water was heated in the throughflow heater (18) to the required brewing temperature.

3. Method according to the preceding claim, **characterised in that** the throughflow heater (18) at the start of a preparation process of a cup measure is preheated before conveying water for heating in step a).

4. Method according to any one of the preceding claims, **characterised in that** additional gas is conveyed during flow through the venturi nozzle in step c).

5. Coffee drinks machine for coffee preparation from substrate capsules, which are insertable into a brewing chamber (20) and comprise a venturi nozzle with a gas feed for producing foam, comprising a throughflow heater (18) for heating water to brewing temperature and a pump (16) for the transport of water from a reserve tank (10) via the throughflow heater (18) to the brewing chamber (20), **characterised by** a device (28) for increasing at least one of the two volume flows (V) of coffee beverage or gas in the venturi nozzle for the duration of a determined time interval, which is determinable in dependence on volume flow, at the end of a cup preparation process.

6. Coffee drinks machine according to claim 5, **characterised by** a control device (28) as a device by which the conveying output of the pump (16) can be increased for the determined duration above an optimum output at which the water is heated to the required brewing temperature.

7. Coffee drinks machine according to one of claims 5 and 6, **characterised by** a conveying device for the feed of gas.

## Revendications

1. Procédé de production de mousse dans un percolateur à café qui comprend un chauffe-eau instantané (18) pour l'échauffement d'eau et qui peut être équipé de capsules de substrat, qui comprend un tube de Venturi pour la production de mousse, avec les étapes suivantes se déroulant en continu :
a) échauffement de l'eau dans le chauffe-eau instantané (18),
b) infusion de mouture de café avec l'eau échauffée dans une chambre d'infusion (20) pour la production d'une boisson au café,
c) production de mousse par mélange de la boisson au café avec un gaz dans le tube de Venturi,
**caractérisé en ce qu'**à l'étape c) au moins un des deux débits volumétriques (V) de la boisson au café ou du gaz dans le tube de Venturi est augmenté pour la durée d'un intervalle de temps déterminé, commandé en fonction du débit, à la fin d'une opération de préparation d'une tasse.

2. Procédé selon la revendication 1, **caractérisé en ce que** le débit de l'eau au-delà d'une capacité optimale, à laquelle l'eau serait échauffée dans le chauffe-eau instantané (18) à la température d'infusion nécessaire, est augmenté.

3. Procédé selon la revendication précédente, **caractérisé en ce que** le chauffe-eau instantané (18) est préchauffé au début d'une opération de préparation d'une portion pour une tasse avant le transport d'eau pour l'échauffement à l'étape a).

4. Procédé selon l'une quelconque des revendications précédentes, **caractérisé en ce que** lors de la traversée du tube de Venturi à l'étape c) du gaz supplémentaire est transporté.

5. Percolateur à café pour la préparation de café à partir de capsules de substrat qui sont insérables dans une chambre d'infusion (20) et comprennent un tube de Venturi avec une amenée de gaz pour la production de mousse, comprenant un chauffe-eau instantané (18) pour l'échauffement d'eau à la température d'infusion, comprenant une pompe (16) pour le transport de l'eau d'un réservoir (10) dans la chambre d'infusion (20) par l'intermédiaire du chauffe-eau instantané (18), **caractérisé par** un dispositif (28) destiné à augmenter au moins un des deux débits volumétriques (V) de la boisson au café ou du gaz dans le tube de Venturi pour la durée d'un intervalle de temps déterminable en fonction du débit volumétrique à la fin d'une opération de préparation d'une tasse.

6. Percolateur à café selon la revendication 5, **caractérisé par** un dispositif de commande (28) en tant que dispositif au moyen duquel le débit de la pompe (16) peut être augmenté pour la durée déterminée au-delà d'une capacité optimale, à laquelle l'eau est échauffée à la température d'infusion nécessaire.

7. Percolateur à café selon l'une quelconque des revendications 5 à 6, **caractérisé par** un dispositif de transport pour l'amenée de gaz.
